# EUROPEAN PATENT SPECIFICATION

(11) **EP 4 003 199 B1**
(45) Date of publication and mention of the grant of the patent: **02.04.2025**
(21) Application number: 19805629.3
(22) Date of filing: 15.11.2019
(51) Int. Cl.: A61B 17/80, A61B 90/00

(54) **ELASTIC PROSTHETICS OF RIBS**
ELASTISCHE PROTHETIK VON RIPPEN
PROTHÈSES ÉLASTIQUES DE CÔTES

(30) Priority: 09.09.2019 UY 38363
(43) Date of publication of application: 01.06.2022
(73) Proprietor: Oromi, Gaston Enrique, Montevideo (UY); Emedical Sociedad Anonima, Montevideo (UY)
(72) Inventor: LOPEZ, Federico, MONTEVIDEO (UY)
(74) Representative: Durán-Corretjer, S.L.P.
(86) International application number: PCT/EP2019/081442
(87) International publication number: WO 2021/047787

(56) References cited:
- EP-A1- 0 014 823
- DE-C1- 3 803 435
- US-A1- 2006 085 000
- US-A1- 2013 338 719
- US-A1- 2015 238 237
- US-A1- 2018 021 074

## Description

### Field of the invention

The present invention is located in the field of therapeutic devices, specifically in the field of devices for replacing all or part of a rib.

### Background of the invention

For the respiratory function to be fulfilled efficiently, the existence of a rib cage with ribs of normal shape and elasticity is essential. The fracture of one or more neighboring ribs can cause the mechanical stability of the costal wall to be lost in such a way that, at the time of inspiration, the area in which the fractures are located instead of expanding is sunk by the action of the negative pressure created in the ribcage while, at the time of expiration, instead of retracting it expands due to an increase in pressure.

This disorder can endanger the life of the patient when the extent of the unstable sector - also called volet - is wide or its mobility, due to lack of rigidity, becomes important.

The situation is similar in the case of surgical operations in which it is necessary to remove an area from the rib cage.

One possibility to improve respiratory efficiency is to use respiratory mechanical assistance, also called internal pneumatic stabilization, by means of an artificial respirator. However, those patients who, due to the length and severity of their fractures must be connected for a prolonged period to a respirator, usually end up with thoracic deformations by welding the ribs in vicious positions. When the fractures are numerous, such deformations can become very pronounced and definitely compromise the patient's work and social life.

Another possibility to improve respiratory efficiency is the use of surgical stabilization, through devices that can solve the problem. In that sense, various solutions have been presented to correct the problems generated by the loss of mechanical stability of the thorax.

One technique to correct the problem has been the use of intramedullary nails. This device consists of a piece of thick stainless steel wire that is introduced through the convex face of the rib a few centimeters from the fracture focus and into the medullary canal of the bone. The fractured ends are faced and the wire is advanced through the medullary canal of the other fragment, causing it to exit through its convex face.

Another technique consists in the use of metallic agrafes. Metal agrafes are thin pieces formed by a narrow sheet that supports a series of open clamps. After facing each of the fractured ends, the clamps are closed and tightened with a special clamp on the healthy bone, thereby fixing the focus of the fracture.

The intramedullary nail technique and the agrafes technique present the problem that both the nails and the agrafes are straight, which causes the rib to lose its normal curvature and elasticity, which affects its function and aesthetic aspects.

Additionally, in elderly patients and especially when they suffer fractures of the anterior third of the rib, these devices cannot be placed because the bone at that level has very little wall and the wall is spongy. Under such conditions, the wires are loose and the agrafes are embedded in the bone (such inconveniences become relevant because the most serious volets are the previous ones and because it is the elderly patients who least tolerate broad rib fractures).

Another technique involves the use of metal splints. Metal splints are simple metal strips that are molded to the shape of the rib, fixed to it by means of ligatures and after a while they must be removed by a new surgical intervention.

The technique based on metal splints has the drawback that it is not possible to obtain a perfect curvature in manual molding and must be removed after a certain time by further intervention. Likewise, treated patients cannot undergo certain diagnostic techniques, such as magnetic resonance imaging, thus being deprived of access to an important diagnostic resource.

In cases of large rib resections, different procedures have been used to replace the excised wall:
- Interewoven with threads of different constitution, created *in situ* during the operative act with or without the addition of prefabricated meshes.
- Mesh and methacrylate sandwiches, the result of the creation of a prosthesis manufactured based on a piece of methyl methacrylate mesh in microspheres mixed with catalyst, which is then covered with a new mesh layer. Once the resulting layer is solidified, a kind of rigid plastron or shell with fabric edges is obtained in which the fixing points are located.

Some surgeons mold synthetic thread, a paste formed by the sandwich is spread over the patient's flank and with this a curved prosthesis is obtained, but of a curvature different from that of the excised wall sector.

Other operators, in order to avoid the damage caused to the patient by the intense heat developed by the exothermic reaction of methacrylate polymerization, manufacture the plastron on a sterile table and in such cases the resulting plastron is flat.

These procedures have proven effective for occlusion of the gap, but both also have drawbacks. The interweaves provide a repair that is often sufficient to save the patient's life, but the intervened wall is left with some permanent instability.

The sandwiches, on the other hand, result in a good repair, but the wall is rigid and with a bulky foreign body that the patient will definitely carry, consisting of a heavy mass of methacrylate in different degrees of polymerization. According to the experience resulting from its use in hip prosthesis surgery, situations of intolerance of the tissues to the methacrylate thus manipulated can also occur.

An improvement to the prior techniques was presented in the UY32513 Invention Patent application. Said application teaches a costal prosthesis of methyl methacrylate polymerized by physical means having the shape of a thin curved sheet and the profile of an angled "C". The device, however, requires the use of a lot of material, is relatively bulky and has a wide profile, which makes it feel comfortable. Additionally, if it is necessary to cut the prosthesis to adjust its length, the use of sterile tools will be necessary. The presence of holes makes it necessary to pierce the rib in order to insert the prosthesis. Cutting the prosthesis with tools generates dust in the operating room.

The placement of the rib on the bone requires perforations, suturing and eventually cutting with sterile tools, which requires considerable operating time, more expenses and greater risk.

Application ES2574759 shows a prosthesis that acts as a support system for the union of fractured ribs.

However, the ES2574759 prosthesis needs to be supported on the bone in almost its entire length, so it does not serve to replace part of the rib and does not serve to splint the bone. The ES2574759 prosthesis must be sutured and glued to the bone so that it tenses. In addition, it uses a fastening system that employs special flanges with a wire-shaped end.

Another form of fractured rib joint is that provided by DePuy Synthes with the MatrixRIB system (https://www.depuysynthes.com/hcp/cmf/products/qs/MATRIX_RIB_Fixation_System?utm_source=Matri xRibContent&utm_medium=Providers). Said system consists of a series of pre-contoured plates, closing screws and splints that allow the fractured rib to be held together, similar to the teachings of ES2574759. However, the problem with the MatrixRIB system is the same as that of ES2574759: the system is made to stabilize and fix the rib and does not serve to replace part of the rib or splint it.

Document US 2006/085000 A1 discloses bone plates for fixing fractured ribs.

Document DE 38 03 435 C1 describes an implant splint for rib fractures made of resorbable material that has grooves to pass threads, also made of resorbable material, to fasten the splint to a fractured rib.

### Summary Description of the Invention

The object of the present application is a prosthesis for replacing all or part of the ribs that may have been removed as a result of thoracic wall surgery. Thus, the prosthesis of the invention can also be used in the reconstruction of the chest wall.

The prosthesis of the invention allows replacing all or part of the rib, reduces operating times, is easily fractional without leaving loose material and does not require any special fastening element.

The prosthesis of the invention has a flexibility similar to that of the natural ribs and does not need removal, that is to say that a new surgical intervention is not necessary to remove the prosthesis.

### Brief description of the figures

A summary description of the figures is given below.
Figure 1 shows the components of a fractured rib.
Figure 2 shows the use of an intramedullary nail in a fractured rib, according to the prior art.
Figure 3 shows a metallic agrafe to be used on a fractured rib, according to the prior art.
Figure 4 shows the application of a similar agrafe to that of Figure 3 on a fractured rib, according to the prior art.
Figure 5 exemplifies the use of an interwoven with threads on a group of fractured ribs, according to the prior art.
Figure 6 exemplifies the use of a mesh-methacrylate sandwich on a group of fractured ribs, according to the prior art.
Figure 7 shows the application of the sandwich of Figure 6, seen from the top side, according to the prior art.
Figure 8 shows a complete prosthesis according to the invention.
Figure 9 shows another complete view of the prosthesis of the invention.
Figure 10 is a view of a section of the prosthesis of the invention from its concave face in which one end of the prosthesis can be seen.
Figure 11 is a view of a section of the prosthesis of the invention from its concave face in which the other end of the prosthesis can be seen.
Figure 12 is a complete view of a prosthesis of the invention from its concave face
Figure 13 is a complete view of a prosthesis of the invention from its convex face, in which the channel for the radiopaque substance is shown.
Figure 14 is a figure that allows to appreciate the warping of the prosthesis of the invention.
Figure 15 is a detail of the face of one of the ends of the prosthesis of the invention.
Figure 16 is a cross section of the prosthesis of the invention.
Figure 17 is a view that allows the prosthesis of the invention to be seen from the convex face.
Figure 18 is another view of the prosthesis of the invention.
Figure 19 shows a prosthesis of the invention that is stabilizing a fractured rib. This embodiment does not belong to the invention.
Figure 20 shows a prosthesis of the invention that is partially replacing a rib.

### Detailed description of the invention

The object of the invention is an individual prosthesis for replacing all or part of a rib, characterized by comprising:
a- a piece curved in the longitudinal direction in the form of an arc, said piece also presenting a curvature in the transverse direction that defines a concave face, the concave face being the face that rests on the rib, and a convex face, the convex face being the face opposite to the concave face, and a warping along the longitudinal axis and
b- two or more transverse grooves located on the convex face that allow the prosthesis fixing elements to be accommodated to the rib, without the need to pierce the rib, and allow the prosthesis to be cut manually

The "concave face" is the face of the curved piece that rests on the rib, once the prosthesis is used.

The "convex face" is the face of the curved piece that faces the outer side of the body, once the prosthesis is used, that is, the face opposite to the "concave face".

By "splinting", not forming part of the invention, it should be understood that the prosthesis allows two parts of a fractured rib to be helo firmly so that the fractured ends are facing and located next to each other to allow the bone to weld. Splinting prevents fractured ends from moving and thus allows proper bone welding.

Preferably, the prosthesis will be constructed in a material that has a stiffness (and therefore also an elasticity) comparable to the natural stiffness (and elasticity) of the ribs, so as to facilitate the necessary bending for a correct support on the outer surface of the rib. "Correct support" means a firm and uniform support against the bone. Thus, the prosthesis behaves similarly to a natural rib during inspiration and expiration movements.

The outer surface of the human rib has curvature on its longitudinal axis and that curvature changes as it goes from the spine to the sternum area. Therefore, the expert will understand that the curved piece must have a warping on its own longitudinal axis, so as to emulate the outer surface of a human rib. This configuration will facilitate the correct support on the outer surface of the rib.

Warping along the longitudinal axis implies that, if the prosthesis rests on a plane over its length, the angle formed between the plane and the line that joins the two transverse ends to the curved piece on the face of one of the ends Longitudinal will be approximately 90 °, so as to emulate the shape of the rib in the area of the spine, while the angle formed between the plane and the line that joins the two transverse ends to the curved piece will gradually decrease along the piece and on the face of the other of the longitudinal ends the angle formed between the plane and the line that joins the two transverse ends will be an acute angle, so as to emulate the shape of the rib in the sternum area. Obviously, the acute angle and therefore warping will depend on the rib for which the prosthesis is applied.

The said angle of approximately 90 ° will preferably be between 85 ° and 95 °. Preferably, it will be an angle of 90 °.

The aforementioned acute angle depends on the rib for which the prosthesis is intended to be applied.

The warping along the axis is then performed so that the prosthesis takes an approximate shape to that of the fractured rib, so that the prosthesis that best fits the shape of a particular rib can be chosen and can be supported correctly over the fractured rib. In other words, warping along the axis approximately emulates the shape of a certain rib or a group of ribs.

The transverse grooves fulfill two functions: they allow the prosthesis fixing elements to be accommodated to the rib and allow the prosthesis to be cut manually, without tools, to adjust it to the desired length.

The presence of grooves allows the prosthesis to be attached to the rib by means of fixing elements and in this way there is no need to pierce the rib. This procedure reduces operating time, which is of fundamental importance in an operation because surgery times are reduced, operating room costs are reduced and less anesthesia is required (because the operating time is reduced). Any known biocompatible fixation means can be used to fix the prosthesis to the rib, and no special fixation means are necessary. For example, suture thread or conventional sterile seals can be used.

In some cases it may be necessary to cut the prosthesis to adjust its length to the desired length. To cut the prosthesis, it will be enough for the surgeon to press with his thumbs on both sides of the chosen groove. The groove will behave like a weak zone that will allow the piece to be cut. This is also relevant in a surgical intervention, because the operating time of the operating room is reduced, sterile tools are not required and no dust is generated.

Preferably, the transverse grooves will be uniformly spaced along the curved piece.

The prosthesis does not require the existence of through holes, which helps to facilitate its fabrication.

Ribs are not all the same: each rib has its own length and curvature. The expert will then understand that the prosthesis should fit the fractured rib, so there will not be a single size of prosthesis. It will usually be convenient to have four or five prostheses for the ribs on the right side and four or five prostheses for the ribs on the left side, especially for emergency situations.

Since the prosthesis has a certain degree of flexibility and elasticity, it is possible to bend it slightly to adapt it to the shape of the rib. For that reason, it is enough with four or five rib prostheses of each side so that it is possible to use any of the prostheses with a particular rib. The expert will not have difficulty choosing the right prosthesis for each case.

Obviously, if necessary, it is possible to manufacture a customized prosthesis for a particular rib, since the manufacturing process is relatively quick. In that case, the warping along the axis will follow exactly the natural warping of the rib, so as to replicate its shape.

Preferably, the prostheses will have an average size of 290mm long, 10mm wide and 3mm thick, with a variation of +/- 30% in any of these dimensions, according to which ribs the prosthesis is directed.

Preferred materials for the construction of the prosthesis are biocompatible polymeric materials. Especially preferred is polyether ether ketone (PEEK). The polyether ether ketone makes it possible to manufacture a prosthesis that has a degree of stiffness comparable to the natural flexibility of the ribs.

Preferably, at least one line of radiopaque material will be included within the body of the prosthesis and throughout its length. This will facilitate your radiological visibility.

Preferably, that line of radiopaque material will be formed by a substance that contains radiopaque material, said substance being incorporated in a channel that travels the curved piece from one end to the other. In those cases in which it is desired to include a radiopaque material, the curved piece will have at each of its longitudinal ends, a hole that connects with the channel that the piece will travel along its entire length. Through one of these holes the radiopaque material will be injected along the entire channel. Preferably, the radiopaque material is included in a resin that flows through the channel and then polymerizes and solidifies. The expert will understand that the curved piece can be constructed with more than one longitudinal channel, so as to have more than one line of radiopaque material.

Examples of radiopaque materials that can be used are barium sulfate and iodinated contrast media, such as iotalamate, ioxaglate or iopamidol.

Preferably, the surface of the concave face will not be flat, but will have relief to facilitate support. Especially preferred is the presence of projections or projections, which not only facilitate support, but allow the tissue to grow around said reliefs and thereby help to keep the prosthesis fixed. The projections may be distributed over the entire surface of the concave face or only part thereof, although it is preferred that the projections be distributed over the entire surface, more preferably that said distribution is uniform.

By "projections or protrusions" it refers to elements that protrude from the surface of the convex face.

Preferably, the projections will have a pyramidal shape. However, the expert will understand that he can give it any other suitable form, such as, for example, conical, cylindrical or striated.

Below are drawings that allow a better understanding of the invention. The drawings should be considered as particular embodiments of the invention, not being in any way limiting thereof.

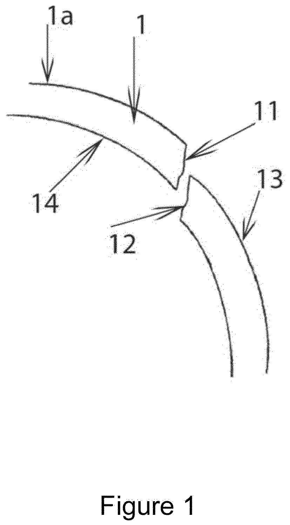

Figure 1 shows a fractured rib **1,** with fractured ends **11, 12.** The rib **1** is then divided into two segments **1a, 1b** due to the fracture. Convex face **13** of the rib and concave face **14** of the rib is shown

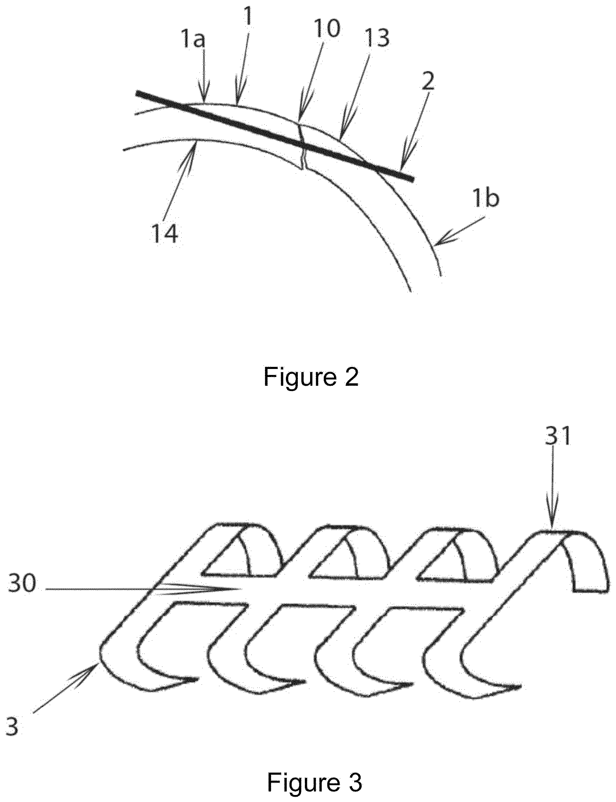

Figure 2 shows the use of an intramedullary nail to repair the fracture, according to the state of the art. In this technique, the nail, which consists of a thick wire **2,** is introduced through the convex face **13** of one of the segments **1b** of the fractured rib **1** a few centimeters from the fracture focus **10** and into the medullary canal of the bone. The fractured ends are faced and the wire **2** is advanced through the medullary canal of the bone until it exits the convex face **13** of the other segment **1a** of the fractured rib **2**
Fig. 3 shows a metallic agrafe **3** according to the state of the art. The agrafe comprises a narrow sheet **30** that supports a series of open clamps **31.** In this case four clamps are shown, but the number could be greater or lesser.

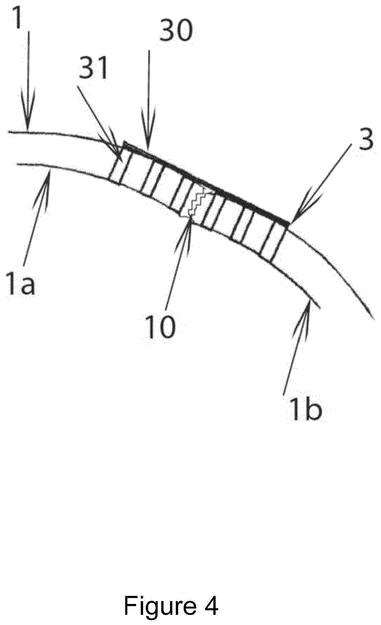

Figure 4 shows the application of the agrafe **3** in a fractured rib 1. The fractured ends are faced, the agrafe 3 is placed and then the clamps **31** are closed and tightened on the healthy bone with a special clamp, thereby fixing the focus of fracture 10.

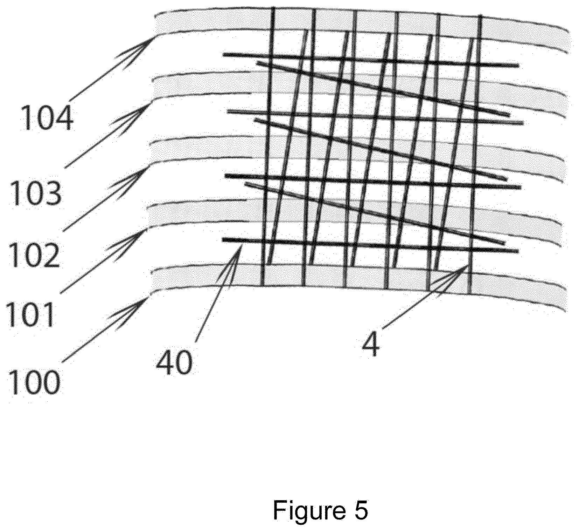

Figure 5 exemplifies the use of an interwoven with threads on a group of ribs **100, 101, 102, 103, 104,** according to the state of the art. The interwoven consists of a thread **4** that joins the different ribs and has threads **40** alternately placed above and below said thread **4** between ribs.

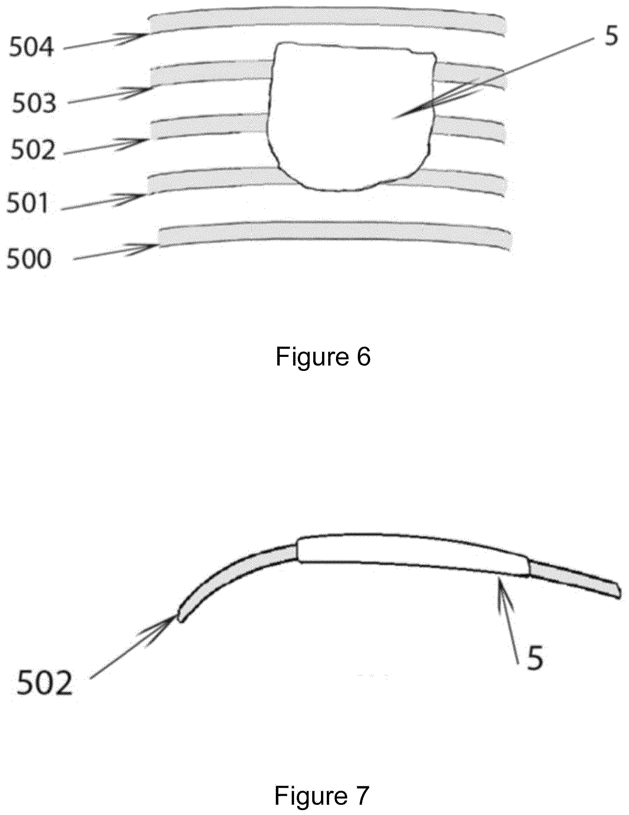

Figure 6 exemplifies the use of a mesh-methacrylate sandwich **5** on a group of ribs **500, 501, 502, 503, 504,** according to the state of the art.

Figure 7 shows the application of the sandwich of Figure **6****,** seen from the top side. A rib 502 and sandwich **5** are seen.

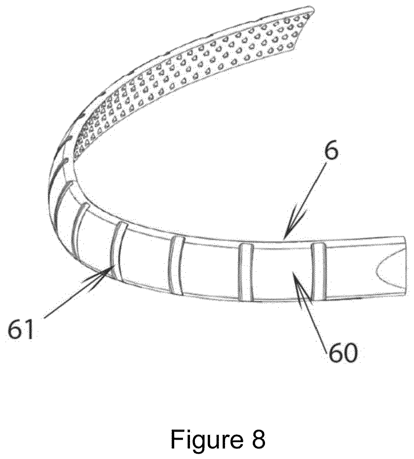

Figure 8 shows a prosthesis **6** according to the invention. The prosthesis comprises a piece 60 longitudinally curved and several grooves **61** in its convex face.

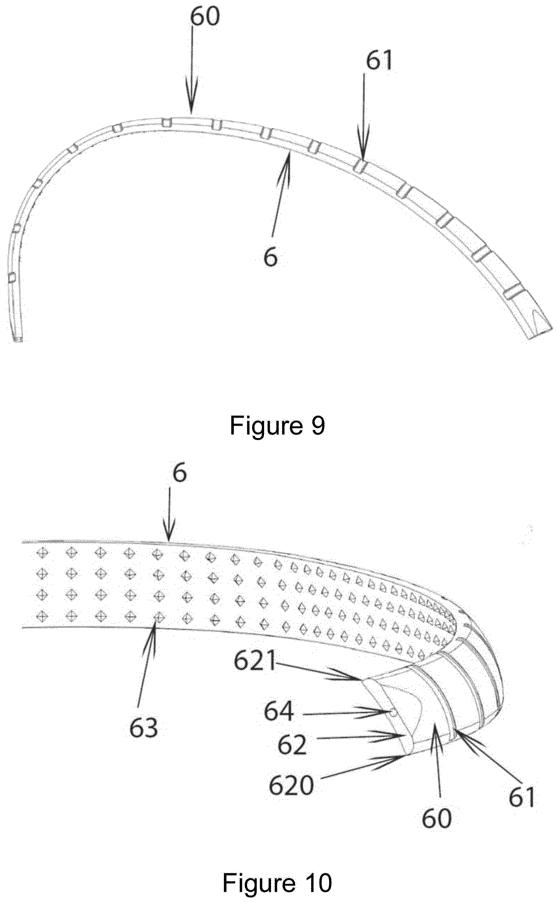

Figure 9 shows another view of the prosthesis **6.** This view shows more clearly the curvature and warping on the longitudinal axis of the piece 60, which emulates the shape of the rib.

Figure 10 is a view of a section of the prosthesis from its concave face and in the area of the end that will be located closer to the sternum. The warping of the piece 60 on its longitudinal axis is appreciated. The face 62 has the two transverse ends to the curved part **620, 621.**

In this case, the piece has projections **63** to improve bone fixation. The number of projections may vary depending on the part or possibly the part may not contain projections.

Also shown on face **62** is the inlet opening **64** to the longitudinal channel intended to contain the opaque radio material. On the other end (not visible in this figure) is the other hole in the channel. In this case, the piece has a single channel, but the expert will understand that there could be more than one channel to place radiopaque material. The expert will also understand that the piece may not contain a channel for radiopaque material: this arrangement makes it more difficult to locate the prosthesis from outside the body, but does not prevent the normal functioning of the prosthesis.

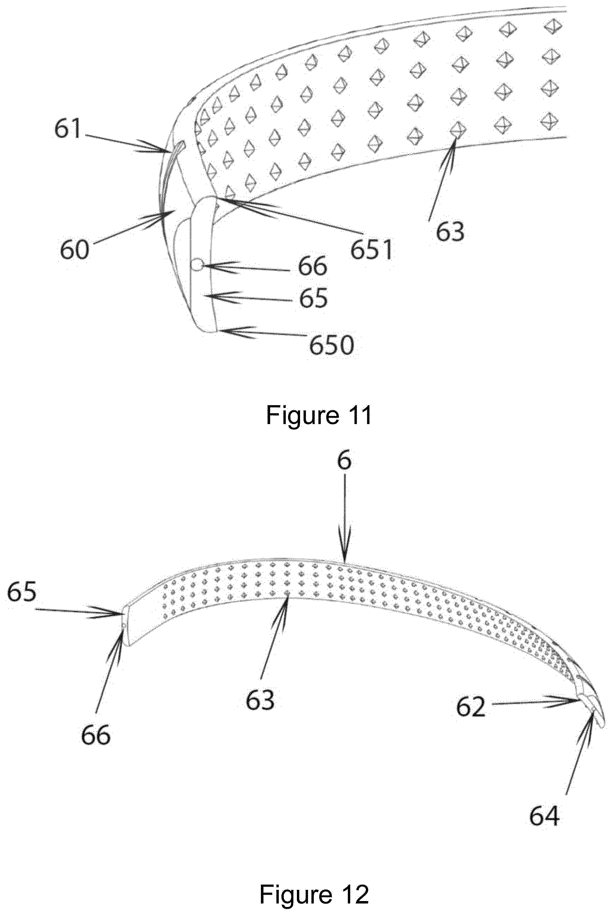

Figure 11 is a view of a section of the prosthesis from its concave face and in the area of the opposite end to that of Figure 10, that is, the end that will be located closer to the spine. In this case it can be seen that the warping of the piece 60 on the longitudinal axis is different from that seen in Figure 10. The face **65** of the other longitudinal end of the prosthesis and the other entrance hole **66** to the channel for the radiopaque material. The face **65** has the two transverse ends to the curved piece **650, 651.**

Figure 12 is a complete view of a prosthesis from its concave face. The two ends **62, 65** and the holes **64, 66** of the entrance to the inner channel are appreciated to locate the radiopaque material. The projections **63** cover partially the concave face.

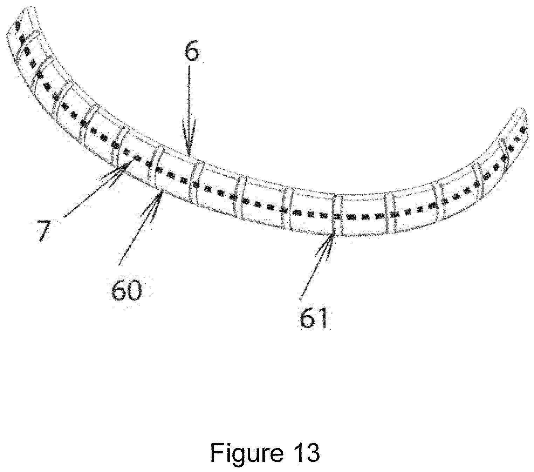

Figure 13 is a complete view of the prosthesis from its convex face. Channel **7** carrying the radiopaque material is shown. The channel 7 is represented with a dotted line to represent that it is internal to the piece **60** and therefore is not visible from the outside (unless the prosthesis does manufacture in some transparent material).

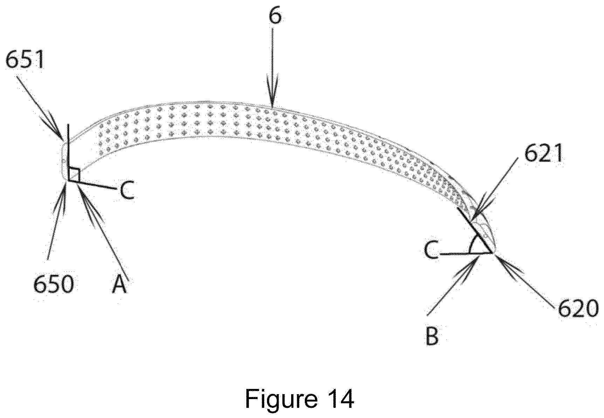

Figure 14 makes it possible to better appreciate the warping of the piece **60.** If the piece **6** is supported on a plane along its entire length, different angles can be seen at the ends. The **C** lines represent the plane on which the prosthesis rests.

At one end, the angle formed between the plane and the line that joins the two ends **650, 651,** belonging to the face **65** and which are transverse to the piece **60,** is an angle A of 90 °. The angle between the plane and the line that joins the two transverse ends varies continuously as you move along the piece **60,** until at the other end the angle formed between the plane and the line that joins the two ends **620, 621,** belonging to the face **62** and which are transverse to the piece **60,** is an acute angle **B.**

This form is convenient because in that way the prosthesis **6** emulates the shape of the rib. The curvature and warping of the piece **60** allows a better support against the convex surface of the rib.

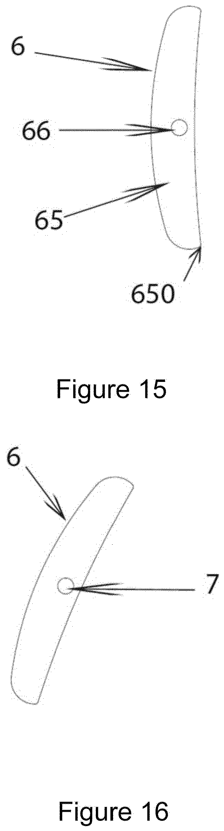

Figure 15 is a detail of the face **65,** in which the orifice **66** of entrance to the channel **7** can be seen. The angle between the line passing through the transverse ends **650, 651** and the support plane is 90 °.

Figure 16 is a section of the piece **60** after advancing a few centimeters in a longitudinal direction from the face **65.** The channel **7** is appreciated and that the angle between the line passing through the transverse ends and the support plane is a smaller angle of 90 °.

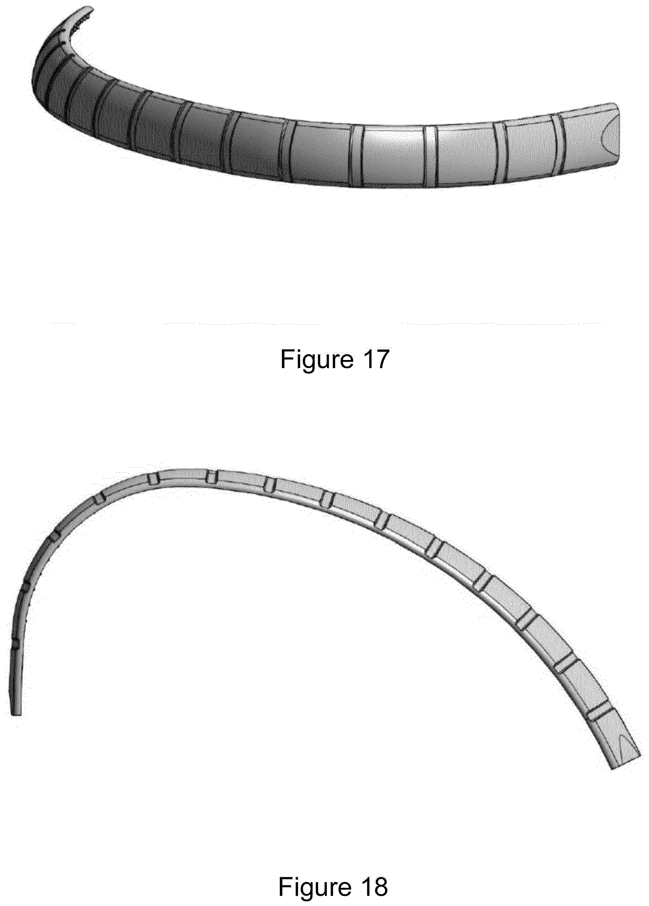

Figure 17 is a view that allows a prosthesis according to the invention to be clearly seen from the convex face.

Figure 18 is another view of the prosthesis of the invention, which allows to see the arch shape of the prosthesis.

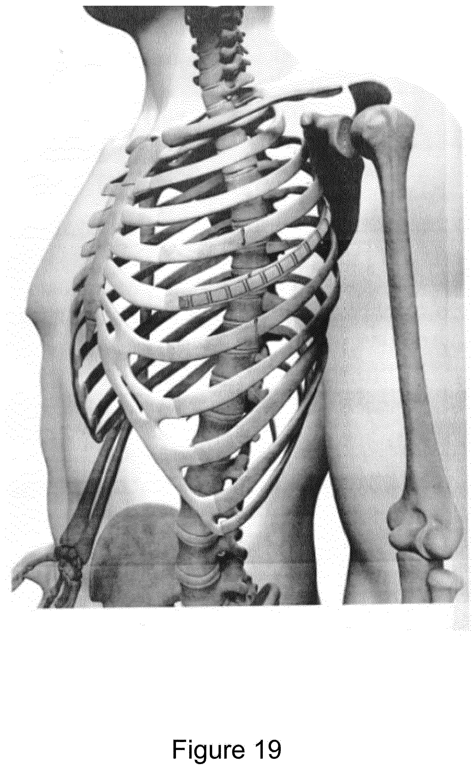

Figure 19 shows a prosthesis, not forming part of the invention, fulfilling the function of splinting a fractured rib. The prosthesis is fixed in this case to the rib by means of suture thread (not shown) that passes through the grooves.

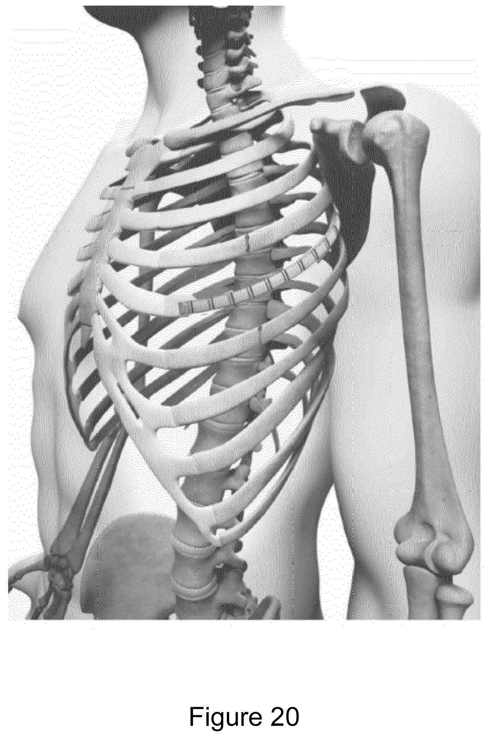

Figure 20 shows the prosthesis of the invention fulfilling the function of partially replacing a fractured rib.

For this, the prosthesis is attached to the ends that remain in the rib and the structure's own rigidity, which is similar to that of the natural rib, allows the structure to behave naturally as if it were part of the rib during the inspiration and expiration movements.

The prosthesis can be manufactured by any known method. For example, it could be manufactured by extrusion with subsequent shaping of the grooves. It could also be manufactured by injection, if it does not carry the channel for the opaque radio material.

A preferred form of manufacturing would be by additive manufacturing (3D printing). Especially preferred is manufacturing by means of molten filament (FFI) or by laser sintering.

Once the prosthesis is manufactured, it is sterilized by any known sterilization method and packaged in sterile form.

As preferred sterilization methods, autoclave sterilization, peroxide sterilization and ethylene oxide sterilization are cited. Especially preferred is ethylene oxide sterilization.

To apply the prosthesis, the prosthesis that best suits the fractured rib is chosen and that will be the one that will rest properly against the bone. Then the prosthesis is adjusted with a suitable means, for example surgical thread, which passes through the grooves and is tied to the bone.

Methods of manufacture, sterilisation and application of the prosthesis do not form part of the invention.

## Claims

1. An individual prosthesis (6) for replacing all or part of a rib, said prosthesis **characterized by** comprising:
a- a piece curved in the longitudinal direction in the form of an arc (60), said piece also presenting a curvature in the transverse direction that defines a concave face, the concave face being the face that rests on the rib, and a convex face , the convex face being the face opposite to the concave face, and a warping along the longitudinal axis and
b- two or more transverse grooves (61) located on the convex face that allow the prosthesis fixing elements to be accommodated to the rib, without the need to pierce the rib, and allow the prosthesis to be cut manually

2. The prosthesis according to claim 1, **characterized in that** the concave face has projections (63).

3. The prosthesis according to claim 1, **characterized in that** the transverse grooves (61) are also spaced apart.

4. The prosthesis according to claim 2, **characterized in that** the transverse grooves (61) are also spaced apart.

5. The prosthesis according to claim 1, **characterized in that** it has at least one line of radiopaque material (7).

6. The prosthesis according to claim 2, **characterized in that** it has at least one line of radiopaque material (7).

7. The prosthesis according to claim 3, **characterized in that** it has at least one line of radiopaque material.

8. The prosthesis according to claim 4, **characterized in that** it has at least one line of radiopaque material (7).

9. The prosthesis according to any of the preceding claims, **characterized in that** the material of said prosthesis is polyether ether ketone.

10. The prosthesis according to claim 9, **characterized in that** it is manufactured by 3D printing.

## Patentansprüche

1. Individuelle Prothese (6) zum vollständigen oder teilweisen Ersatz einer Rippe, **dadurch gekennzeichnet, dass** die Prothese umfasst:
a) ein in Längsrichtung bogenförmig gekrümmtes Teil (60), wobei das Teil auch eine Krümmung in Querrichtung aufweist, die eine konkave Fläche definiert, die die Fläche ist, die auf der Rippe ruht, und eine konvexe Fläche definiert, die die Fläche ist, die der konkaven Fläche gegenüberliegt, sowie eine Verformung entlang der Längsachse, und
b) zwei oder mehr Rillen (61), die sich auf der konvexen Fläche befinden und es ermöglichen, die Prothesenbefestigungselemente ohne die Notwendigkeit, die Rippe anzustechen, an der Rippe anzubringen und die Prothese manuell zuzuschneiden.

2. Prothese nach Anspruch 1, **dadurch gekennzeichnet, dass** die konkave Fläche Vorsprünge (63) aufweist.

3. Prothese nach Anspruch 1, **dadurch gekennzeichnet, dass** die Rillen (61) ebenfalls voneinander beabstandet sind.

4. Prothese nach Anspruch 2, **dadurch gekennzeichnet, dass** die Rillen (61) ebenfalls voneinander beabstandet sind.

5. Prothese nach Anspruch 1, **dadurch gekennzeichnet, dass** sie mindestens eine Linie aus strahlenundurchlässigem Material (7) aufweist.

6. Prothese nach Anspruch 2, **dadurch gekennzeichnet, dass** sie mindestens eine Linie aus strahlenundurchlässigem Material (7) aufweist.

7. Prothese nach Anspruch 3, **dadurch gekennzeichnet, dass** sie mindestens eine Linie aus strahlenundurchlässigem Material aufweist.

8. Prothese nach Anspruch 4, **dadurch gekennzeichnet, dass** sie mindestens eine Linie aus röntgendichtem Material (7) aufweist.

9. Prothese nach einem der vorstehenden Ansprüche, **dadurch gekennzeichnet, dass** das Material der Prothese Polyetheretherketon ist.

10. Prothese nach Anspruch 9, **dadurch gekennzeichnet, dass** sie durch 3D-Druck hergestellt ist.

## Revendications

1. Prothèse individuelle (6) pour le remplacement de tout ou partie d'une côte, ladite prothèse étant **caractérisée en ce qu'**elle comprend :
a- une pièce courbée dans la direction longitudinale en forme d'arc (60), ladite pièce présentant également une courbure dans la direction transversale qui définit une face concave, la face concave étant la face qui repose sur la côte, et une face convexe, la face convexe étant la face opposée à la face concave, et une déformation le long de l'axe longitudinal et
b- deux ou plusieurs rainures transversales (61) situées sur la face convexe qui permettent de loger les éléments de fixation de la prothèse sur la côte, sans qu'il soit nécessaire de percer la côte, et de couper manuellement la prothèse.

2. Prothèse selon la revendication 1, **caractérisée en ce que** la face concave présente des saillies (63).

3. Prothèse selon la revendication 1, **caractérisée en ce que** les rainures transversales (61) sont également espacées.

4. Prothèse selon la revendication 2, **caractérisée en ce que** les rainures transversales (61) sont également espacées.

5. Prothèse selon la revendication 1, **caractérisée en ce qu'**elle a au moins une ligne de matériau radio-opaque (7).

6. Prothèse selon la revendication 2, **caractérisée en ce qu'**elle a au moins une ligne de matériau radio-opaque (7).

7. Prothèse selon la revendication 3, **caractérisée en ce qu'**elle a au moins une ligne de matériau radio-opaque.

8. Prothèse selon la revendication 4, **caractérisée en ce qu'**elle a au moins une ligne de matériau radio-opaque (7).

9. Prothèse selon l'une quelconque des revendications précédentes, **caractérisée en ce que** le matériau de ladite prothèse est une polyéther-éther-cétone.

10. Prothèse selon la revendication 9, **caractérisée en ce qu'**elle est fabriquée par impression 3D.
